(19) Europäisches Patentamt
European Patent Office
Office européen des brevets

(11) **EP 4 276 195 A1**

(12) **EUROPEAN PATENT APPLICATION**

(43) Date of publication:
**15.11.2023 Bulletin 2023/46**

(21) Application number: **22172653.2**

(22) Date of filing: **10.05.2022**

(51) International Patent Classification (IPC):
***C12Q 1/6827*** (2018.01)      ***C12Q 1/6883*** (2018.01)

(52) Cooperative Patent Classification (CPC):
(C-Sets available)
**C12Q 1/6827; C12Q 1/6883**                (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Genartis S.r.l.
37126 Verona (IT)**

(72) Inventors:
• **DELLEDONNE, Massimo
37134 Verona (IT)**
• **ROSSATO, Marzia
30036 S. Maria di Sala (IT)**

(74) Representative: **Zaccaro, Elisabetta et al
Notarbartolo & Gervasi S.p.A.
Viale Achille Papa, 30
20149 Milano (IT)**

Remarks:
The complete document including Reference
Table(s) and the Sequence Listing(s) can be
downloaded from the EPO website

(54) **METHODS FOR DETERMINING THE PRESENCE OR RISK OF DEVELOPING MYOTONIC DYSTROPHY TYPE 1 (MD1) OR MYOTONIC DYSTROPHY TYPE 2 (MD2)**

(57)     The present invention concerns the field of repeat expansion-linked disorder, and in particular to an in-vitro method for determining whether said subject is suffering from or has a genetic predisposition to develop such medical conditions.

The invention further relates to uses of isolated oligonucleotides and a kit carrying out Cas9-mediated sequencing or PCR-free targeted long-read sequencing to be applied in translational research and in clinical settings, with ultimately strong benefits for the diagnostic workflow and genetic counselling in this class of diseases. Further described in the present invention is a method for the validation of the cut efficiency a guideRNA.

EP 4 276 195 A1

(52) Cooperative Patent Classification (CPC): (Cont.)

C-Sets
**C12Q 1/6827, C12Q 2521/301, C12Q 2522/101, C12Q 2537/159;
C12Q 1/6883, C12Q 2521/301, C12Q 2522/101, C12Q 2537/159**

**Description**

FIELD OF THE INVENTION

The present invention concerns the field of repeat expansion-linked disorder, and in particular to an in-vitro method for determining whether said subject is suffering from or has a genetic predisposition to develop such medical conditions.

The invention further relates to uses of isolated oligonucleotides and a kit carrying out long-DNA capture approaches and sequencing to be applied in translational research and in clinical settings, with ultimately strong benefits for the diagnostic workflow and genetic counselling. Further described in the present invention is a method for the validation of the cut efficiency of a guide-RNA (gRNA).

STATE OF THE ART

Myopathies are diseases that affect muscles that control voluntary body movements, and that can be experienced by patients as muscle weakness, due to a dysfunction of the muscle fibers which are weakened by the disease.

Myopathies can have a genetic origin and can therefore be passed on in families (inherited) the most common of which are muscular dystrophies, or they may develop later in life (acquired) due to autoimmune disease, toxic myopathy, endocrine myopathy, metabolic disorders or other causes.

Diagnosing a myopathy and determining the specific myopathy from which a patient suffers can require many tests and exams.

When the physician suspects a myopathy, the first step of the medical diagnosis is that of acquiring the patient's medical history and proceeding with an objective neurological examination and by performing blood tests for determining creatine phosphokinase (CPK), a cytoplasmic enzyme specific for evaluating muscle cell condition and by performing a dosage of inflammatory indices.

If these parameters are high, the diagnosis is directed towards an inflammatory muscle disease (polymyositis and dermatomyositis), which will be confirmed by a muscle biopsy.

If, on the other hand, these parameters are normal, an electromyographic examination (EMG) is performed, which is the gold standard to distinguish between neurogenic or myogenic alterations, giving the physician a precise picture of the patient's nerve conduction.

Neurogenic myopathies can be due to peripheral nerve diseases (diagnosed by peripheral nerve biopsy or genetic tests) or to diseases of the motion neuron (Amyotrophic Lateral Sclerosis - clinically diagnosed - or Spinal Muscular Atrophy - diagnosed with genetic examination).

In the case of myogenic alterations, the specialist needs to evaluate the myotonic phenomenon, which is useful for assessing whether there is a defect in the muscle's relaxation process. In the most frequent cases, the myotonic phenomenon is not present and a muscle biopsy is performed, which can allow to diagnose many different diseases.

If, on the other hand, the myotonic phenomenon is present (it is the less frequent situation), genetic investigations are performed to diagnose between different myopathies such as myotonic dystrophy type I (DM1, which involves the DMPK gene) or myotonic dystrophy type II (DM2, involving the CNBP gene) or other diseases with myotonia such as Canalopathies, involving the genes such as CLCN1, SCN4A, CACNA1S, KCNJ2, KCNJ5.

Molecular diagnosis of DM1 and DM2 has been seen to be extremely challenging, due to the fact that the DMPK and the CNBP genes have very large microsatellite expansions in the disease state. The extreme length of the microsatellite repeats (from 75 to >11.000 units in the CNBP gene) and the strong gene mosaicism deeply challenge the molecular diagnosis of DM1 and DM2 patients and hampered the sequencing of fully expanded alleles so far.

Presently the best practice for DM1 and DM2 genetic testing relies mainly on PCRbased approaches. These include a first step where short-range PCR (SR-PCR) is used to exclude the diagnosis of DM1 and DM2 when two alleles within the normal range are detected. When only one allele is visible, nucleotide repeat expansions can be identified either with Long Range PCR (LR-PCR) or Quadruplets-Repeat Primed PCR (QP-PCR), leading to around 99% of detection rate. Despite enabling DM1 or DM2 diagnosis, both methods do not allow to define the exact length of the large expanded alleles, which can only be determined using Southern blot on digested genomic DNA, with a sensitivity of about 80%. Furthermore, this procedure is timeconsuming, requires large amount of DNA and it is not included in the routine workflow of most diagnostic centers. Moreover, the currently used methods completely lack single-nucleotide resolution, important especially to determine repeat interruptions, and have severe limitations in detecting the presence of minor alleles and the level of somatic mosaicism, all aspects known to be possible modulators of disease phenotype and severity.

The need and importance are increasingly felt for a more rapid diagnostic method, which allows for the molecular diagnosis of repeat expansion disorders in a precise and effective manner, which can easily be implemented in the normal diagnostic workflow in standard laboratories and avoid the limitations discussed above for the currently used standard methods. A deep novel targeted-long read sequencing method, enabling the analysis of clinically relevant repeats, is still lacking.

The object of the present invention is therefore the development of a diagnostic method based on genetic analysis that requires a single biological sample, for example a blood test, for proceeding with DNA extraction and analysis. The method allows, with a single test, to characterize pathogenic loci and to diagnose myopathies that involve DNA expansion, and other repeat expansion disorders in an efficient way.

SUMMARY OF THE INVENTION

The present invention concerns a method for the diagnosis of pathologies harboring repetitive elements. The analysis of such genomic features and defects is of utmost importance as they underlie numerous congenital disorders, in particular neurological pathologies and myopathies. The method of the present invention enables a more precise genotype-phenotype correlation and patient stratification in clinical trials for personalized therapeutic approaches.

The method of the present invention is a PCR-free Cas9-mediated sequencing and allows the characterization, at single-nucleotide resolution of the disease-underlying genes.

As it will be further described in the detailed description of the invention, in a first aspect the present invention relates to an in-vitro method for diagnosing a repeat expansion-linked disorder in a human subject, said method comprising the steps of:

    a. providing a biological sample comprising genomic DNA from said subject;
    b. extracting genomic DNA from the biological sample, preferably said genomic DNA is high-molecular-weight DNA (>50kbp);
    c. analysing the portion of the genomic DNA in said sample by Cas9-mediated DNA enrichment and by long-read DNA sequencing,

wherein said repeat expansion-linked disorder is chosen from the group consisting of Myotonic dystrophy type 1 (MD1) and Myotonic dystrophy type 2 (MD2), and wherein said method allows to determine whether said subject is suffering from or has a genetic predisposition to develop Myotonic dystrophy type 1 or from Myotonic dystrophy type 2.

In a second aspect the invention relates to the use of an isolated oligonucleotide guideRNA for diagnosing a repeat expansion-linked disorder chosen from the group consisting of Myotonic dystrophy type 1 and Myotonic dystrophy type 2 in a human subject, wherein:

MD1 is diagnosed by analysing the portion of the genomic DNA in the sample corresponding to the DMPK gene on chromosome 19q13.32 and determining the presence or absence of a CTG repeat expansion in the 3'-UTR of the DMPK gene with at least one, preferably two guideRNAs chosen from the group consisting SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10; and MD2 is diagnosed by analysing the portion of the genomic DNA in the sample corresponding to the CNBP gene on chromosome 3q21.3 and determining the presence or absence of a CCTG or TCTG repeat expansion in the intron 1 of the CNBP gene with at least one, preferably two guideRNA chosen from the group consisting of SEQ ID NO: 11 and SEQ ID NO: 12.

In a third aspect the invention herein describes a kit for diagnosing a repeat expansion-linked disorder chosen from the group consisting of Myotonic dystrophy type 1 and Myotonic dystrophy type 2 in a human subject, said kit comprising:

    at least one, preferably two guideRNAs chosen from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10; and/or
    at least one, preferably two guideRNAs chosen from the group consisting of SEQ ID NO: 11 and SEQ ID NO: 12 and instructions for use in the method of the invention.

In a fourth aspect, the present invention relates to a method for the validation of the cut efficiency a guideRNA comprising the steps of:

    a. selecting a guideRNA to be validated;
    b. subjecting a sample of genomic DNA to Cas9-mediated cleavage using the selected guideRNA of step a., to obtain a cleaved DNA (Cut DNA);
    c. amplifying the cleaved DNA (Cut DNA) of step b. with a primer pair surrounding the gRNA cut site (Target gene) and in parallel amplifying the cleaved DNA (Cut DNA) of step b. with a second primer pair amplifying a genomic region unaffected by Cas9 cleavage (Control Cut DNA);

d. amplifying a sample of genomic DNA (Uncut DNA) with the primer pair surrounding the gRNA cut site (Target gene) of step b. and in parallel amplifying a sample of genomic DNA (Uncut DNA) with the second primer pair of step b. (Control Uncut DNA);

e. validating the cut efficiency of the guideRNA by applying formula (I):

$$Fold\ change = \frac{primer\ Eff.\ Target\ gene\ ^\wedge\ (Ct\ Uncut\ DNA\ -\ Ct\ Cut\ DNA)}{primer\ Eff.\ Control\ gene\ ^\wedge\ (Ct\ Control\ Uncut\ DNA\ -\ Ct\ Control\ Cut\ DNA)}$$

wherein the Cut efficiency of each gRNA (i.e. % of cut DNA) is determined using formula (II):

$$Cut\ efficiency = (1 - Fold\ change)\ x\ 100$$

wherein a cut efficiency value of preferably more than or equal to 70% is indicative that a guideRNA has been validated.

The formula calculates the relative change (Fold Change) of detection (Ct, Cycle threshold) in Cut vs Uncut DNA of the Target gene over the level of detection of the Control gene. The formula takes into consideration also the efficiency of amplification of each primer pair (primer Eff.).

BRIEF DESCRIPTION OF THE DRAWINGS

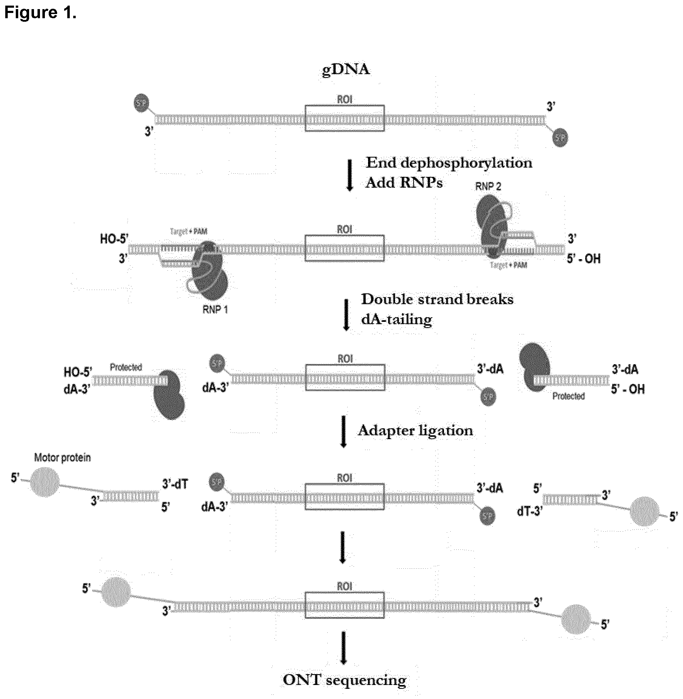

The characteristics and advantages of the present invention will be apparent from the detailed description reported below, from the Examples given for illustrative and nonlimiting purposes, and from the annexed Figures 1-4:

**Figure 1. Schematic representation of the Cas9-mediated target sequencing workflow.** The Cas9-mediated targeted sequencing method employs the specificity of RNPs (target specific guideRNA, comprising a crRNA combined with tracerRNA, and complexed with Cas9 enzyme) for the targeted excision of genomic regions and sequencing using the (Oxford Nanopore Technology) ONT long-read platform. Briefly, starting gDNA is dephosphorylated to prevent downstream unwanted adapter ligation. Two RNP complexes are formed, one on each side of the target, consisting of a gRNA (crRNA + tracrRNA targeting the region of interest) and the Cas9 nuclease. Upon target recognition, RNPs-mediated cleavage originates 5'-phosphorylated blunt-ended DNA. The incorporation of a non-templated dAMP on the 3'-end of cleaved gDNA (dA-tailing) facilitates the specific ligation of the ONT sequencing adapters containing the motor protein. The final library includes also non-target fragments which, in principle, cannot be sequenced since they lack the 5'-phosphate and cannot be ligated by the ONT adapters. The whole library is loaded into the ONT sequencing platform and the enrichment is provided by the sequencing itself, owing to the fact that only target fragments bear sequencing adapters.

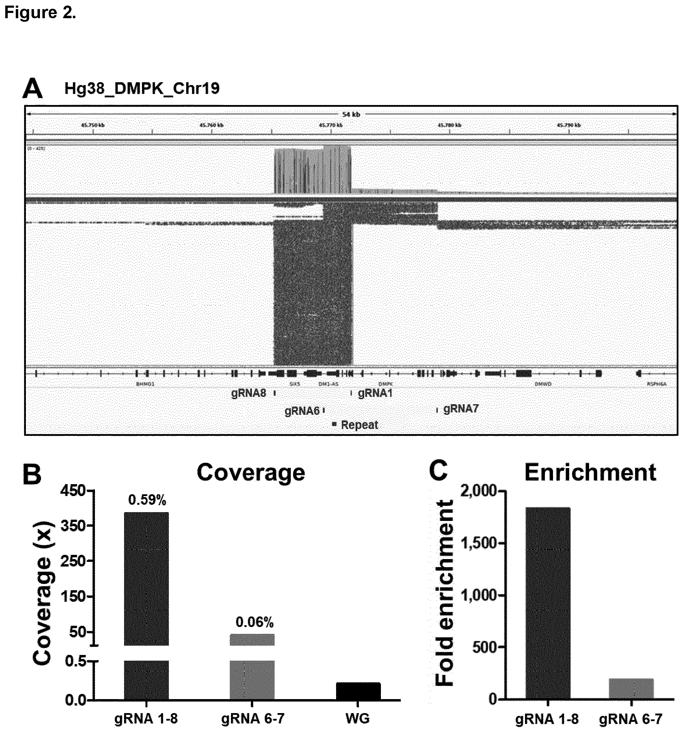

**Figure 2. Cas9-mediated enrichment and ONT sequencing of the DMPK microsatellite as described in Example 1. (A)** Integrative Genomics Viewer (IGV) visualization of ONT reads on the DMPK microsatellite comprising the DMPK repeat in the 3'UTR. The position of the gRNAs and the repeat are indicated at the bottom. Both the enriched targets (gRNA1-8 and gRNA6-7) displays clear cuts on both sides, tough to different degrees. **(B)** Average coverage data on the DMPK microsatellite and whole genome (WG, i.e. background coverage). Values on top indicate the % of on-target reads. **(C)** Fold enrichment of the DMPK microsatellite for the two gRNA couples.

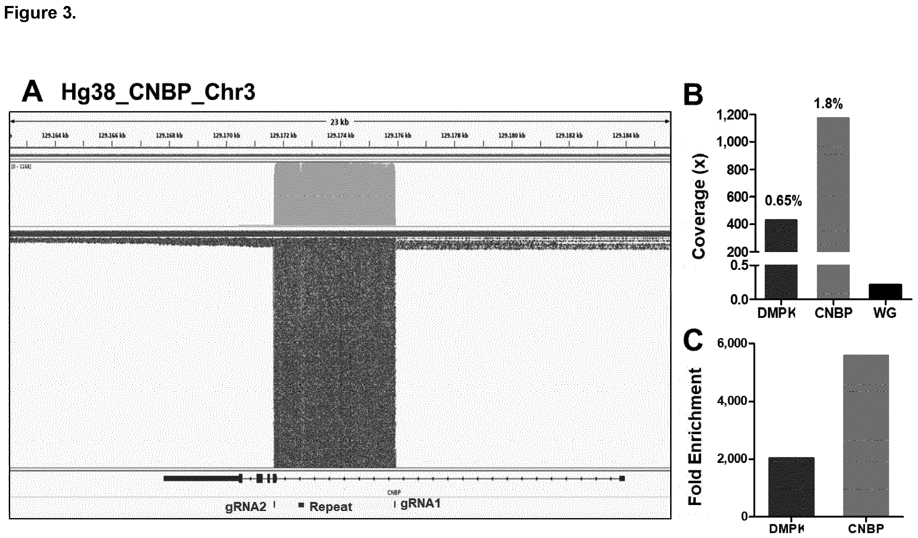

**Figure 3. Cas9-mediated enrichment and ONT sequencing of the CNBP microsatellite as described in Example 1. (A)** Integrative Genomics Viewer (IGV) visualization of ONT reads mapping on the CNBP microsatellite, comprising the CNBP repeat in intron 1. The position of the gRNAs and the repeat are indicated at the bottom. The enriched target displays clear cuts on both sides, accounting for a good performance of Cas9-mediated capture. **(B)** Average coverage data at the CNBP microsatellite and at whole genome level (WG, i.e. background coverage). Values on top indicate the % of on-target reads. **(C)** Fold enrichment data of the CNBP microsatellite. For comparison, statistics on the DMPK microsatellite Cas9-mediated sequencing are also shown.

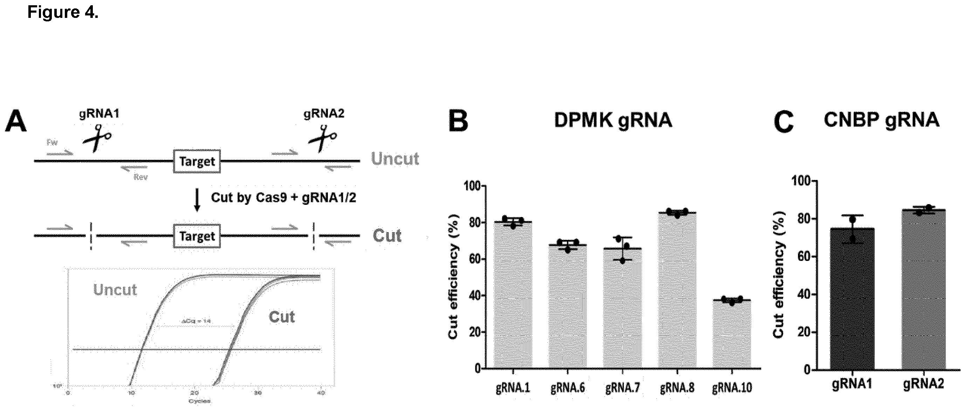

**Figure 4: Evaluation of gRNA cut efficiency by qPCR as described in Example 2. (A)** Schematic representation of the qPCR assay used to evaluate the cut efficiency of the gRNAs. Primer pairs were designed flanking each gRNA cut site and then used to amplify intact or cut DNA, respectively. The amount of intact versus cut DNA is compared and plotted as cut efficiency (i.e. % of cut DNA). In all analysis, PRL (non-target gene) was used for DNA input normalization. **(B)** Cut efficiency of gRNA designed for Cas9-mediated sequencing of the DMPK microsatellite. The gRNAs were pooled together and used to simultaneously cut genomic DNA (N=3). **(C)** Cut efficiency of gRNA designed for Cas9-mediated sequencing of the CNBP microsatellite. The gRNAs were pooled together and used to simultaneously cut genomic DNA (N=2).

DETAILED DESCRIPTION OF THE INVENTION

Pathogenic loci can include tandem repeats, namely sequences of two or more DNA base pairs that are repeated adjacent to each other. The current assembly of the human genome (Hg38) has been reported to contain over one million of annotated tandem repeats. They are usually found in non-coding regions of the genome, even though short repetitions of triplets are frequently localized also in coding portions. Their intrinsic repetitive nature makes tandem repeats particularly prone to mutations. They bear indeed the highest mutational rate in the genome and are typically polymorphic and multiallelic, with the longest alleles being the most unstable. However, interruptions of the canonical repeat by alternative repeated motives have been shown to stabilize the expansions, conferring milder and/or different phenotypes compared to uninterrupted ones. Tandem repeat expansions are the causative phenomenon of at least 50 known human disorders. Expansions affecting coding regions usually cause the formation of polyAlanine (PolyA), polyGlutamine (PolyQ) or polyGlycine (polyG) stretches. These may lead either to protein loss of function(polyA) or to protein (polyQ) / peptide (polyG) toxicity. Expansions affecting non-coding portions are usually found either in 5'-untranslated regions (5'-UTRs), introns or 3'-UTRs. Noncoding repeats in 5' regions usually lead to hypermethylation and subsequent gene silencing. Intronic expansions and expansions affecting 3' UTRs are more often linked to RNA toxicity or polypeptide synthesis via repeat-associated non-AUG (RAN) translation and subsequent formation of cellular/nuclear aggregates. A common characteristic of repeat expansion disorders is genetic anticipation, namely a phenomenon in which the signs and symptoms of some genetic conditions tend to become more severe and/or appear at an earlier age as the disorder is passed from one generation to the next. The mechanism underlining repeat expansion is attributable to the difficulties encountered by the DNA replication machinery within long stretches of repeated DNA. Repeated units seem to be deleted or added to long repetitive tracts as the cellular machinery tries to replicate through DNA hairpins formed by repeated sequences.

Genes known to bear repeat expansions include DMPK (DM1 protein kinase) and CNBP (CCHC-type zinc finger nucleic acid binding protein). Expansions in these genes lead to Myotonic dystrophy type 1 (DMPK, DM1) and Myotonic dystrophy type 2 (CNBP, DM2), respectively. In particular, DM1 and DM2 patients have been reported to bear some of the longest expansions, up to 19.5 kbp (DM1) and 44 kbp (DM2). The present invention concerns in a first aspect, an in-vitro method for diagnosing a repeat expansion-linked disorder in a human subject, said method comprising the steps of:

a. providing a biological sample comprising genomic DNA from said subject;
b. extracting genomic DNA from the biological sample, preferably said genomic DNA is high-molecular-weight DNA (>50kbp);
c. analysing the portion of the genomic DNA in said sample by Cas9-mediated DNA enrichment and by long-read DNA sequencing,

wherein said repeat expansion-linked disorder is chosen from the group consisting of Myotonic dystrophy type 1 (MD1) and Myotonic dystrophy type 2 (MD2), and wherein said method allows to determine whether said subject is suffering from or has a genetic predisposition to develop Myotonic dystrophy type 1 or from Myotonic dystrophy type 2.

The diagnostic method of the present invention can be promoted to a more widespread application for the characterization of also other pathogenic loci, only partially resolved using traditional approaches.

The method of the present invention could allow patient stratification and enable a more precise genotype-phenotype correlation which is clinically beneficial for the patient in order to have a tailored medical and pharmaceutical assistance and the most efficient clinical trials.

In a preferred aspect, in the in-vitro method according to the invention, said step of long-read DNA sequencing is carried out by nanopore sequencing (i.e. ONT-sequencing) or SMRT sequencing (i.e. PacBio) or other long-read sequencing technologies at single nucleotide resolution. The in-vitro method of the invention is a PCR-free method. One of the advantages of long-DNA capture methods is in fact represented by the possibility to perform PCR-free assays, thus escaping potential biases related to this type of amplification, such as in regions with extreme CG content and repetitions. Potentially there are no limitations on target length, as soon as good quality, HMW gDNA is provided and the gRNA design is performed properly.

For the purposes of the present invention, the term "guideRNA" or the abbreviation "gRNA", intends to comprise a piece of RNA that functions as a guide for RNA- or DNAtargeting enzymes with which the guideRNA forms a complex. GuideRNAs can be designed to be used for targeted DNA editing, such as with CRISPR-Cas9 and CRISPR-Cas12. A Cas9 guideRNA is composed by two part, namely the tracrRNA and the crRNA, that form base pairs with DNA target sequences, enabling Cas9 to introduce a site-specific double-strand break in the DNA. The tracrRNA provides structural support to the crRNA whereas the latter provides target recognition based on complementarity to the genome.

For ease of reference and according to the general practice, the crRNA sequences reported in the present document are generally called "guideRNA" or "gRNA" even if they miss the tracrRNA component. For their efficient use, "guideRNA" or "gRNA" reported in the document must be complexed with the tracrRNA, as "two-part" guideRNA or syntethized as "single guide RNA" comprising both the crRNA sequence and the tracrRNA. In the case a "two-part" guideRNA is selected the synthetic crRNA must contain a sequence enabling the complementarity with the tracrRNA as described in Example 1.

As indicated herein, "isolated guideRNAs" are in-vitro synthetized. It is also advisable that synthetic guideRNA are chemically modified to enhance RNA stability.

The term "Cas9-mediated sequencing", intends to comprise Cas9-mediated enrichment combined with long-read sequencing, a method which allows to assess the length and structure of both normal and expanded alleles, and to recognize the presence of repeat interruptions and mosaicism, as well as DNA modifications such as methylation.

In a preferred aspect, in the in-vitro method according to the invention, said step of Cas9-mediated enrichment is carried out by Cas9 enzyme, that can be replaced with other suitable Cas enzymes, e.g. Cas12, with adequate sequencing protocol adjustments.

In addition, the possibility to exploit a PCR-free assay enables the unbiased, comprehensive characterization of complex genomic regions, including epigenetic modifications of DNA such as methylation.

Most of the known repeat expansion disorders are of recent discovery, probably due to the limitations of the current approaches used in diagnosis. Best practice guidelines for the diagnosis involve a first step where short-range PCR (SR-PCR) is used to assess whether an individual has two alleles with a low number of repeats. If only one allele is detected, pathogenic expansions can be addressed via long-range PCR (LR-PCR) or repeat-primed PCR (RP-PCR) on genomic DNA (gDNA) or LR-PCR products. However, the presence of interruptions may result in aberrant patterns or failure to detect expansions with RP-PCR. In such cases, Southern blotting of LR-PCR products or gDNA is usually performed for confirmation. However, this procedure is timeconsuming, requires large amount of DNA and it is not included in the routine workflow of most diagnostic centers. These methods are often simultaneously required for reliable diagnosis. Moreover, they can be imprecise when dealing with extremely long expansions, mosaicism and minor alleles. Above all, they lack single-nucleotide resolution which is crucial for the accurate characterization of both the repeat structure, e.g. interruptions, and mosaicism level, known to be disease modulators.

NGS (Next Generation Sequencing) approaches provide an unprecedented opportunity for the characterization of repeat expansion at single-nucleotide resolution. In this context, the combination of long-read sequencing with long-DNA capture approaches provides a valuable tool for the comprehensive characterization of repeat expansions, which are often associated to pathologic conditions. Long reads can span across the entire repeated region, including their flanking sequences of higher complexity, and provide a single nucleotide resolution. This enables more accurate mapping providing insights on repeat length and structure, and presence of specific features such as interruptions and mosaicism. Moreover, the possibility to map methylation simultaneously to sequencing data acquisition provides an added value to the analysis and the possibility to correlate this molecular feature to the pathological phenotype. Overall, a more accurate characterization of repeat-features would lead to a more precise genotype-phenotype correlations for repeat expansion disorders, that is still lacking/limited for those disease with large microsatellite expansions, such as DM1 and DM2, probably due to the limitations of current diagnostic approaches.

As disclosed herein, in the in-vitro method according to the invention, wherein said Myotonic dystrophy type 1 is due to an expansion of a microsatellite locus within the DMPK (DM1 protein kinase) gene, whereas said Myotonic dystrophy type 2 is due to an expansion of a microsatellite locus within the CNBP (CCHC-type zinc finger nucleic acid binding protein) gene.

Repeat expansion in the DMPK gene (MIM*605377) is causative of Myotonic dystrophy type 1 (DM1; MIM#160900), an autosomal dominant disorder characterized mainly by myotonia, muscular dystrophy, cataracts, hypogonadism, frontal balding, and ECG changes. The disease is caused by a (CTG)n repeat expansion in the 3'-UTR of the DMPK gene on chromosome 19q13.32. The protein encoded by DMPK is a serine-threonine kinase which, among others, has the role to inhibit the muscle protein myosin phosphatase, which in turn plays a role in muscle tensing and relaxation. Expanded alleles produce altered mRNAs containing double-stranded hairpin structures, which are not translated and form clumps with other proteins in the cytoplasm. Among these we find Muscleblind Like Splicing Regulator 1 (MBNL1), which is a protein involved in RNA splicing. The depletion of its activity leads to impaired splicing disrupts muscle development and function. Non-pathogenic alleles contain up to 36 CTG-repeat units, whereas pre-mutated allele contain between 37 and 49 repeats. In DM1 patients, repetitions range from 50 to up to 6,500 units in congenital forms. Expanded repeats are highly unstable and lead to show genetic anticipation. Moreover, the CTG tract has been shown to expand during an individual's lifetime, resulting in somatic mosaicism and the worsening of the disease symptoms with age. In 3-5 % of the DM1 patients the CTG tract of expanded alleles is interrupted by non-CTG tracts such as CCG, CTC or GGC motives. Importantly, these so-called variant repeats have been reported to stabilize the repeated tract with significant implications in disease onset and progression. As a matter of fact, patients with variant repeats may exhibit delayed onset, unusually mild symptoms, or atypical patterns of symptoms.

Repeat expansion in the CNBP gene (previously ZNF9, MIM*116955) is causative of Myotonic dystrophy type 2 (DM2; MIM#602668), an autosomal dominant multisystemic disorder characterized by progressive proximal muscle weakness, myotonia, myalgia, calf hypertrophy and multiorgan involvement with cataract, cardiac conduction defects and endocrine disorders. The disease is caused by a (CCTG)n repeat expansion in intron 1 of the CNBP gene on chromosome 3q21.3. CNBP encodes for a singlestranded DNA-binding protein which is essential for embryonic development in mammals. Nevertheless, still little is known about its function and molecular pathways, though it seems to be involved in cytoplasmic post-transcriptional gene regulatory processes rather than acting as transcription factor. The pathogenic mechanism of DM2 is similar to DM1 and involves the formation of alternative mRNAs containing double-strand hairpin structures which in turn sequester cytoplasmic proteins involved in RNA splicing such as MBNLs. This leads to impaired splicing disrupts muscle development and function. The CCTG repeat tract is part of a complex (TG)v(TCTG)w(CCTG)x motif (SEQ ID NO: 13) which is generally interrupted in healthy range alleles by one or more GCTG, TCTG or ACTG motifs resulting in repeat stability. Non-pathogenic alleles contain up to 26 CCTG-repeat units, whereas premutations are made of "pure" (CCTG)<75 with still uncertain clinical significance. In DM2 patients, repetitions range between -75 and >11,000 units and represent some of the largest reported so far in repeat expansion disorders. The DM2 mutation shows marked somatic instability and tends to increase in length over time within the same individual, while it does not show a strong bias towards intergenerational expansion and genetic anticipation is rarely seen in DM2 families. The few genotype-phenotype studies reported so far in DM2 patients did not reveal any significant associations between the severity of the disease, including the age at onset, and the number of CCTG repeated units. Identification of such correlations is indeed strongly challenged by heterogeneity across tissues, somatic instability, and the relative technical difficulty of accurately measuring repeat length in such large microsatellite expansions. For the same reasons, the discovery of additional cis genetic modifiers that may protect or exacerbate disease symptoms is hampered.

The genetic features of the CNBP microsatellite locus along with its extreme length and high CG-content hampered the ability to sequence expanded alleles in DM2 patients. Indeed, investigators of the original gene-discovery study were unable to sequence the entire CCTG array because of its extremely large size and the high level of somatic mosaicism.

In a still preferred aspect, in the in-vitro method according to the invention, said biological sample comprising genomic DNA is chosen from the group consisting of fetal cells obtained by amniocentesis, fetal cells obtained by chorionic villus sampling, biopsy (for example from muscle, bone marrow, nerve etc.), blood, plasma, serum, urine, saliva and tears.

In a further aspect, in the in-vitro method according to the invention, said step c. of analysing the portion of the genomic DNA in the sample by Cas9-mediated DNA enrichment and by long-read DNA sequencing is carried out by analysing the portion of the genomic DNA in the sample corresponding to the DMPK gene on chromosome 19q13.32 and determining the presence or absence of a CTG repeat expansion in the 3'-UTR of the DMPK gene, wherein a determination of the absence of a CTG repeat expansion in the 3'-UTR of the DMPK gene indicates that the subject does not have a genetic predisposition to develop, and is not suffering from Myotonic Dystrophy type 1, and/or wherein a determination of the presence of a CTG repeat expansion in the 3'-UTR of the DMPK gene indicates that the subject has a genetic predisposition to develop, or is suffering from Myotonic Dystrophy type 1 (MD1).

In a still further aspect, in the in-vitro method according to the invention, said step c. of analysing the portion of the genomic DNA in the sample by Cas9-mediated DNA enrichment and by long-read DNA sequencing is carried out by analysing the portion of the genomic DNA in the sample corresponding to the CNBP gene on chromosome 3q21.3 and determining the presence or absence of a CCTG or TCTG repeat expansion in the intron 1 of the CNBP gene, wherein a determination of the absence of a CCTG or TCTG repeat expansion in the intron 1 of the CNBP gene indicates that the subject does not have a genetic predisposition to develop, and is not suffering from Myotonic Dystrophy type 2, and/or wherein a determination of the presence of a CTG repeat expansion in the 3'-UTR of the CNBP gene indicates that the subject has a genetic predisposition to develop, or is suffering from Myotonic Dystrophy type 2 (MD2).

An increase in the presence or amount of the DMPK repeat provides a clinical diagnosis of MD1, whereas the presence or amount of the CNBP repeat provides a clinical diagnosis of MD2.

In some embodiments, an increase in the repeat amount over a certain threshold of the DMPK or CNBP repeats is indicative of disease presence or predisposition.

In one embodiment, the reference standard is the level of the one or more DMPK or CNBP repeats measured in one or more biological sample(s) obtained from healthy control subjects known not to have MD1 or MD2. One or more healthy individuals can be used to generate a reference standard for use in the methods.

In a further embodiment, the reference standard are reference values as indicated above for the DMPK gene, where non-pathogenic alleles contain up to 36 CTG-repeat units, and for the CNBP gene where non-pathogenic alleles contain up to 26 CCTG-repeat units.

Application of the Cas9-mediated sequencing in the clinical setting as per the diagnostic method of the present invention can improve molecular diagnosis, genetic counselling and genotype-phenotype correlation, which are still lacking/limited for those disease characterized by large microsatellite expansions.

The Cas9-mediated targeted sequencing method employs the specificity of RNPs (target specific guideRNA, comprising a crRNA combined with traduced with tracerRNA, and complexed with Cas9 enzyme) for the targeted excision of genomic regions and sequencing using the (Oxford Nanopore Technology) ONT long-read platform. **(Figure** 1). Briefly, starting gDNA is dephosphorylated to prevent downstream unwanted adapter ligation. Two RNP complexes are formed, one on each side of the target, consisting of a gRNA (crRNA + tracrRNA targeting the region of interest) and the Cas9 nuclease. Upon target recognition, RNPs-mediated cleavage originates 5'-phosphorylated blunt-ended DNA. The incorporation of a non-templated dAMP on the 3'-end of cleaved gDNA (dA-tailing) facilitates the specific ligation of the ONT sequencing adapters containing the motor protein. The final library includes also non-target fragments which, in principle, cannot be sequenced since they lack the 5'-phosphate and cannot be ligated by the ONT adapters. The whole library is loaded into the sequencing platform and the enrichment is provided by the sequencing itself, owing to the fact that only target fragments bear sequencing adapters.

In a preferred aspect, in the in-vitro method according to the invention, said step c. of analysing the portion of the genomic DNA in the sample by Cas9-mediated sequencing comprises contacting the sample with a guideRNA that specifically hybridizes to at least a portion of the region corresponding to nucleotides chr19:45,745,000-45,799,000 (hg38), which encompasses a portion of the DMPK locus in the 19q13.32 band.

In a preferred aspect, in the in-vitro method according to the invention, said step c. of analysing the portion of the genomic DNA in the sample by Cas9-mediated sequencing comprises contacting the sample with the guideRNA selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10.

In a preferred aspect, in the in-vitro method according to the invention, said step c. of analysing the portion of the genomic DNA in the sample by Cas9-mediated sequencing comprises contacting the sample with a guideRNA that specifically hybridizes to at least a portion of the region corresponding to nucleotides chr3:129,163,000-129,185,000 (hg38), which encompasses a portion of the CNBP locus in the 3q21.3 band.

In a preferred aspect, in the in-vitro method according to the invention, said step c. of analysing the portion of the genomic DNA in the sample by Cas9-mediated sequencing comprises contacting the sample with the guideRNA selected from the group consisting of: SEQ ID NO: 11 and SEQ ID NO: 12

In a second aspect the invention relates to the use of an isolated oligonucleotide guideRNA for diagnosing a repeat expansion- linked disorder chosen from the group consisting of Myotonic dystrophy type 1 and Myotonic dystrophy type 2 in a human subject, wherein:

MD1 is diagnosed by analysing the portion of the genomic DNA in the sample corresponding to the DMPK gene on chromosome 19q13.32 and determining the presence or absence of a CTG repeat expansion in the 3'-UTR of the DMPK gene with at least one, preferably two guideRNAs chosen from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10 and
MD2 is diagnosed by analysing the portion of the genomic DNA in the sample corresponding to the CNBP gene on chromosome 3q21.3 and determining the presence or absence of a CCTG or TCTG repeat expansion in the intron 1 of the CNBP gene with at least one, preferably two guideRNAs chosen from the group consisting of SEQ ID NO: 11 and SEQ ID NO: 12.

In a preferred aspect of the use for diagnosing a repeat expansion-linked disorder according to the invention, combinations of 2 guideRNAs cutting at each side of the repeat and at >3 kbp of distance from each other are selected, preferably more than or equal to 500bp on each side.

In a third aspect the invention herein describes a kit for diagnosing a repeat expansion-linked disorder chosen from the group consisting of Myotonic dystrophy type 1 and Myotonic dystrophy type 2 in a human subject, said kit comprising:

at least one, preferably two guideRNAs chosen from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10; and/or
at least one, preferably two guideRNAs chosen from the group consisting of SEQ ID NO: 11 and SEQ ID NO: 12 and instructions for use in the method of the invention.

In a fourth aspect, the present invention relates to a method for the validation of the cut efficiency a guideRNA comprising the steps of:

a. selecting a guideRNA to be validated;
b. subjecting a sample of genomic DNA to Cas9-mediated cleavage using the selected guideRNA of step a., to obtain a cleaved DNA (Cut DNA);

c. amplifying the cleaved DNA (Cut DNA) of step b. with a primer pair surrounding the gRNA cut site (Target gene) and in parallel amplifying the cleaved DNA (Cut DNA) of step b. with a second primer pair amplifying a genomic region unaffected by Cas9 cleavage (Control Cut DNA);

d. amplifying a sample of genomic DNA (Uncut DNA) with the primer pair surrounding the gRNA cut site (Target gene) of step b. and in parallel amplifying a sample of genomic DNA (Uncut DNA) with the second primer pair of step b. (Control Uncut DNA);

e. validating the cut efficiency of the guideRNA by applying formula (I):

$$Fold\ change = \frac{Eff.\ Target\ gene \wedge (Ct\ Uncut\ DNA - Ct\ Cut\ DNA)}{Eff.\ Control\ gene \wedge (Ct\ Control\ Uncut\ DNA - Ct\ Control\ Cut\ DNA)}$$

The formula calculates the relative change (Fold Change) of detection (Ct, Cycle threshold) in Cut vs Uncut DNA of the Target gene over the level of detection of the Control gene. The formula takes into consideration also the efficiency of amplification of each primer pair (primer Eff.). Cut efficiency of each gRNA (i.e. % of cut DNA) was then determined using the following formula (II):

$$Cut\ efficiency = (1 - Fold\ change)\ x\ 100$$

wherein a cut efficiency value of more than or equal to 70% is indicative that the selected guideRNA has been validated. For "primer pair surrounding the gRNA cut site (Target gene) of step b." it is intended to comprise the region containing the gRNA cut site.

The guideRNA set is validated when it has a cutting efficiency value, which means that the percentage of Cut DNA is preferably more than or equal to 70% of the total, more preferably more than 70%.

The choice of the primer pair depends on the target gene and on the gRNA cut site. It is essential that a primer pair surrounds the cut site of the selected gRNA (Target gene), i.e. whose derived-amplicon comprises the guideRNA cleavage site; in parallel an uncut target site (control gene, e.g. PRL gene) is amplified at the purpose of data normalization.

As it will be further described in the Examples, primer pairs flanking each gRNA cut site were manually designed and are reported in **Table 2.** The application of the method allowed to validate the cut efficiency of guide RNA, setting a standard for the validation of further gRNAs, which are independent of the gene locus.

It was demonstrated that a cut efficiency of more than or equal to (≥) 70% was indicative of obtaining an efficient guide RNA that can be used in Cas9-mediated capture and ONT sequencing.

The method for the guideRNA cut efficiency validation of the invention has the advantages of being simple and fast and can be routinely carried out in a standard molecular biology laboratory, allowing to obtain and study many samples and gRNAs simultaneously. When compared to the costs and benefits of using a standard sequencing procedure, the method of the invention is cost- and time-effective and provides effective and optimized guideRNAs which are adaptable to different aims, including Cas9-mediated sequencing and also genome editing.

For the purposes of the present disclosure, each guideRNA has a corresponding SEQ ID NO. as indicated in **Table 1** where genomic coordinates refer to the human reference genome assembly hg38:

Table 1: GuideRNA sequences and guideRNA target sequences

| Target | gRNA ID | guideRNA | | guideRNA target sequence | | PAM | Chr | [Start] | [End] | Stran d |
|---|---|---|---|---|---|---|---|---|---|---|
| | | SEQ ID NO | guideRNA sequence | SEQ ID NO | guideRNA target sequence | | | | | |
| DMPK | gRNA1_ DMPK | 1 | CGGACAACCAG AACUUCGCC | 13 | CGGACAACCAG AACTTCGCC | AGG | 19 | 457717 73 | 45771 792 | - |
| DMPK | gRNA6_ DMPK | 2 | UCGAGCUUGC GUCCCAGGAG | 14 | TCGAGCTTGCG TCCCAGGAG | CGG | 19 | 457693 76 | 45769 395 | + |
| DMPK | gRNA7 _ DMPK | 3 | GGUCUUCAGG AAUUCUAACG | 15 | GGTCTTCAGGA ATTCTAACG | GGG | 19 | 457789 72 | 45778 991 | - |
| DMPK | gRNA8_ DMPK | 4 | AGUCCCCCAC GUAUAUGGCA | 16 | AGTCCCCCACG TATATGGCA | GGG | 19 | 457652 42 | 45765 261 | + |
| DMPK | gRNA10 _DMPK | 5 | UCGAUCUUUG AGCUGAGCGC | 17 | TCGATCTTTGA GCTGAGCGC | TGG | 19 | 457724 05 | 45772 424 | - |
| DMPK | gRNA14 _DMPK | 6 | UUCCCUGGCC CAAGUUUCGG | 18 | TTCCCTGGCCC AAGTTTCGG | TGG | 19 | 457672 36 | 45767 255 | + |
| DMPK | gRNA15 _DMPK | 7 | ACCAAUGUCG GGAGAGGCCA | 19 | ACCAATGTCGG GAGAGGCCA | CGG | 19 | 457654 66 | 45765 485 | + |
| DMPK | gRNA16 _DMPK | 8 | CUGGUCAUGG AGUAUUACGU | 20 | CTGGTCATGGA GTATTACGT | GGG | 19 | 457786 19 | 45778 638 | - |
| DMPK | gRNA17 _DMPK | 9 | CGUCCACUACA AGGUGAGCA | 21 | CGTCCACTACA AGGTGAGCA | CGG | 19 | 457776 60 | 45777 679 | - |
| DMPK | gRNA18 _DMPK | 10 | GUGUAUAGACA CCUGGAGGA | 22 | GTGTATAGACA CCTGGAGGA | GGG | 19 | 457714 82 | 45771 501 | - |

EP 4 276 195 A1

| Target | gRNA ID | guideRNA | | guideRNA target sequence | | PAM | Chr | [Start] | [End] | Stran d |
|---|---|---|---|---|---|---|---|---|---|---|
| | | SEQ ID NO | guideRNA sequence | SEQ ID NO | guideRNA target sequence | | | | | |
| CNBP | gRNA1_ CNBP | 11 | CCACCUGAUUC ACUGCGAUA | 23 | CCACCTGATTC ACTGCGATA | GGG | 3 | 129175 929 | 12917 5948 | - |
| CNBP | gRNA2_ CNBP | 12 | GGCUUCUCAU UCCACGACCA | 24 | GGCTTCTCATT CCACGACCA | CGG | 3 | 129171 664 | 12917 1683 | + |

Various embodiments and aspects of the present invention as delineated hereinabove and as claimed in the claims section below find experimental support in the following examples.

EXAMPLES

Reference is now made to the following examples, which together with the above descriptions illustrate some embodiments of the invention.

**Example 1: Cas9-mediated enrichment coupled to ONT sequencing for the analysis of DMPK and CNBP repeats.**

Genomic DNA extraction. High molecular weight (HMW) genomic DNA (gDNA) from the human embryonic kidney 293 (HEK293) cell line was extracted starting from cell pellets (~1 million) using the Genomic-tip 100/G Kit (Qiagen) based on manufacturer's instructions. DNA was resuspended with Tris-EDTA buffer pH 8. DNA quantity was measured using the QuBit Fluoromether (ThermoFisher Scientific) in combination with the Qubit dsDNA BR Assay Kit (ThermoFisher Scientific), while integrity was assessed using TapeStation 4150 (Agilent, Santa Clara, California, USA) in combination with Genomic DNA ScreenTape (Agilent) and Genomic DNA Reagents (ladder and sample buffer, Agilent).

Cas9-mediated enrichment. Design of crRNAs was conducted using the online tool CHOPCHOP (https://chopchop.cbu.uib.no/) following Oxford Nanopore (ONT)'s recommendation (https://community.nanoporetech.com/info sheets/tarqetedamplification-free-dna-sequencing-using-crispr-cas/v/eci s1014 v1 reve 11dec2018). gRNA designed upstream the target repeat were complementary to the reverse DNA strand, while gRNA designed downstream the target repeat were complementary to the forward strand. gRNAs were designed at >500bp from the target repeats and combined in couples to enrich and sequence >3kbp fragments. Candidate gRNAs were manually checked for unique mapping via alignment on the human genome (Hg38) using BLAST and excluding region overlapping common SNPs (MAF > 0.01, dbSNP database). The following gRNA couples have been selected for the experiments shown hereby: gRNA6_DMPK/gRNA7_DMPK or gRNA1_DMPK/gRNA8_DMPK (**Figure** 2) or gRNA1_CNBP/gRNA2_CNBP (**Figure** 3), the gRNA sequences are reported in **Table** 1. The gRNAs were purchased from Integrated DNA Technologies (Custom Alt-R® CRISPR-Cas9 guide RNA, IDT, Coralville, Iowa, USA) and used at 10 $\mu$M together with transactivation crRNA (Alt-R synthetic tracrRNA, 10 $\mu$M, IDT) in the presence of 62 $\mu$M Alt-R® S.p. HiFi Cas9 Nuclease V3 (IDT) in 1X CutSmart Buffer for the generation of Ribonucleoprotein (RNP) complexes according to the ONT protocol (https://community.nanoporetech.com/info sheets/tarqeted-amplification-free-dna-sequencinq-usinq-crispr-cas/v/eci s1014 v1 reve 11dec2018). DNA dephosphorylation, Cas9-mediated digestion and dA-tailing were performed based on the same ONT protocol. Briefly, Input gDNA, 1 - 10 $\mu$g, was dephosphorylated using 15 units of Quick Calf Intestinal Phosphatase (New England Biolabs) in 1X CutSmart Buffer (New England Biolabs) for 10' at 37°C and then 2' at 80°C for enzyme inactivation. RNPs were incubated with gDNA 20' at 37°C and then 2' at 80°C for enzyme inactivation. 10 $\mu$l of pre-assembled RNPs were mixed to dephosphorylated DNA for target cleavage and simultaneous dA-tailing with dATP, using 5 units of Taq DNA polymerase (New England Biolabs) and 10 mM dATP (New England Biolabs). Cas9-mediated digestion and dA-tailing were performed 20' at 37°C and 5' at 72°C to inactivate the Cas9. Five $\mu$l of ONT's sequencing adapters (AMX, ONT) were ligated for 10' at RT to the cleaved, dA-tailed DNA using 20 $\mu$l Ligation Buffer (ONT) and 10 $\mu$l of Quick T4 DNA ligase from the NEBNext® Quick Ligation Module (New England Biolabs). The reaction was stopped by adding 1 volume of 10 mM Tris-EDTA pH 8. Short fragments < 3 kbp and residual enzymes were removed by adding 0.3X AMPure XP beads (Beckman-Coulter, Brea, California, USA) and by washing twice using the Long fragment buffer (LFB, ONT). DNA was eluted 10' at RT using the Elution Buffer (EB, ONT). Multiple samples can be sequenced simultaneously using the Cas9-multiplexing protocol. Briefly, Cas9-multiplexing experiments were performed as described above with the exception that native barcodes (EXP-NBD104, ONT) were ligated to the cleaved and dA-tailed target ends using Blunt/TA Ligase Master mix (New England Biolabs). Samples were quantified and all available nanograms were pooled in a final volume of 65 $\mu$l nuclease-free water. Ligation of ONT sequencing adapters (AMXII from EXP-NBD104, ONT), purification, washing steps and elution were performed as previously described for the singleplex experiments.

ONT sequencing. Purified library was mixed with 37.5 $\mu$l of Sequencing buffer (ONT) and 25.5 $\mu$l of Library loading beads (ONT). Library was loaded on a FLO-MIN106D (R9.4.1) flow cell and sequenced using MinKNOW (ONT, v20.06.5) until plateau was reached.

ONT sequence data analysis. Base-calling of raw fast5 files was conducted using Guppy v3.4.5 with high-accuracy mode, with parameters "-r -i $FAST5_DIR -s $BASECALLING_DIR --flowcell FLO-MIN106 --kit SQK-LSK109 --pt_scaling TRUE". The latter parameter was suggested by ONT tech support to perform the most appropriated scaling of reads with biased sequence composition, as expected with low complexity repeat motifs. Reads from multiplexed runs were demultiplexed with Guppy v3.4.5 with parameters "-i $BASECALLING_DIR -s $DEMuITIPLEXING_DIR --trim_barcodes --barcode_kits EXP-NBD104". Reads were quality filtered with NanoFilt v2.7.133, requiring a minimum quality score of 7. Reads spanning the full repeat were identified using a combination of msa.sh and cutprimers.sh scripts from BBMap suite v38.87 (https://sourceforge.net/projects/bbmap/). In particular, identification was conducted by in-silico PCR and using 100 bp primers placed on the repeat's flanking regions (minimum alignment identity: 80%). These "complete sequences" were extracted and used for the subsequent analyses. Complete sequences were assigned either to the wild-type or to the expanded allele based on their length, based on k-means clustering method (k = 2). Reads from each allele and each sample were then processed separately.

An accurate consensus sequence was obtained collapsing reads from the wild-type allele as described in Grosso et al., 2021 (doi: 10.3389/fgene.2021.743230), specifying medaka "r941_min_high_g344" model. The polished consensus sequence was finally searched for repeats using Tandem Repeat Finder v4.0935. Scripts for generating consensus sequences and repeats annotations for the normal allele are reported in https://github.com/MaestSi/CharONT.

Reads from the expanded allele were aligned to sequences flanking the repeat, searched for repeats with motif "CTG" (DMPK) or "TG", "CCTG" and "TCTG" (CNBP), and visualized in a genome browser, as described in Grosso et al., 2021. In particular, extracted sequences and the repeat summary file were then imported in R and used for generating a simplified version of the reads with a custom script. In particular, reads coordinates corresponding to an annotated repeat were replaced by a single nucleotide stretch of length equaling the annotated repeat, with each motif corresponding to a different nucleotide stretch. The flanking region was reported unchanged to enable simplified reads alignment, while "TG" was replaced with "GG", "CCTG" with "CCCC", "TCTG" with "TTTT", "CTG" with "AAAA" and remaining nonannotated portions were replaced with stretches of "N" of the same length. Simplified reads which were originally in reverse orientation were reverse complemented. All simplified reads were aligned to the flanking sequence with Minimap2 v2.17-r941 and visualized in IGV v2.8.3. Scripts for annotating repeats and generating simplified reads for the expanded allele are reported in https://github.com/MaestSi/MosaicViewer_CNBP.

Results from the DMPK locus. Cas9-mediated sequencing of the DMPK microsatellite comprising the repeat in the 3'UTR was performed as described above combining gRNA 1_DMPK with gRNA 8_DMPK and gRNA 6_DMPK with gRNA 7_DMPK **(Figure 2A)**. To prevent any cut interference between gRNAs on the same end of the target, cut reactions were performed in parallel and then pooled prior to ONT sequencing. The assay was performed on gDNA extracted from the HEK293 cell line, known to carry not-expanded alleles (http://hek293genome.org/v2/). gRNA pair 1-8 produced 385 reads (0.59%) and gRNA pair 6-7 40 reads (0.06%) on target **(Figure 2B),** corresponding to 1,833-fold and 190-fold enrichment, respectively **(Figure** 2C).

The *de novo* assembly of reads derived from the DMPK microsatellite locus, performed as described above, showed that the HEK293 cells carry two different alleles with the structure $(CTG)_{14}$ (SEQ ID NO: 25) and $(CTG)_5$ (SEQ ID NO: 26), and falling into the normal repeat range, thus demonstrating that the Cas9-mediated repeat analysis was successful and returned correct results in the assessment of allele type.

Results from the CNBP locus. Cas9-mediated sequencing of the CNBP microsatellite comprising the repeat in intron 1 was performed as described above combining gRNA 1_CNBP with gRNA2_CNBP to enrich a fragment of 4.1 kbp **(Figure 3A).** The assay was performed on gDNA extracted from the HEK293 cell line, known to carry not-expanded alleles (http://hek293genome.org/v2/) and including gRNA pair 1-8 for DMPK as internal control. ONT sequencing performed after Cas9-mediated enrichment generated a total of 65,154 PASS reads, of which 1,595 (2.45%) were on-target. The gRNA pairs designed on CNBP generated clear cuts on both sides of the target **(Figure 3A)** and provided up to 1,170 (1.8%) on-target reads **(Figure 3B),** confirming the successful Cas9-mediated sequencing. Whole genome coverage was 0.21x, resulting in a 5,571-fold enrichment of the CNBP microsatellite **(Figure 3C).** The *de novo* assembly of reads derived from the CNBP microsatellite locus, performed as described above, showed that the HEK293 cells carry two identical and undistinguishable alleles with the structure $(TG)_{20}(TCTG)_{10}(CCTG)_6(GCTG)_1(CCTG)_1(TCTG)_1(CCTG)_7$ (SEQ ID NO: 27), and falling into the normal "Short Interrupted Allele Type B", thus demonstrating that the Cas9-mediated repeat analysis was successful and returned correct results in the assessment of allele type.

**Example 2: Analysis of gRNA cut efficiency by qPCR Analysis**

Quantitative PCR (qPCR) analysis. Primer pairs flanking the gRNA cut site of gRNA1_DMPK, gRNA6_DMPK, gRNA7_DMPK, gRNA8_DMPK, gRNA10_DMPK, gRNA1_CNBP and gRNA2_CNBP were manually designed and are reported in Table 2, where genomic coordinates refer to the human reference genome assembly hg38.

Table 2: Primer sequences utilized in Example 2

| SEQ ID NO: | ID | Primer | Sequence | Chromosome | [Start] | [End] | Amplicon length (bp) | Efficiency |
|---|---|---|---|---|---|---|---|---|
| 28 | gRNA1_DMPK | Fw | CTGAAGTGGCAGTTCCAGCG | 19 | 45.771.908 | 45.771.927 | 275 | 1,03 |
| 29 | | Rev | CTCGCGTAGTTGACTGTGGG | | 45.771.653 | 45.771.672 | | |
| 30 | gRNA6_DMPK | Fw | CACCGAGCGTCGAGAAGAGG | | 45.769.462 | 45.769.481 | 132 | 0,88 |
| 31 | | Rev | GGCTAGAAAGTTTGCAGCAACTT | | 45.769.350 | 45.769.372 | | |
| 32 | gRNA7_DMPK | Fw | AGGGTGTGTCAGGTGGATGAG | | 45.779.108 | 45.779.128 | 187 | 1,02 |
| 33 | | Rev | CCCAGACACTCCTTCCCTAG | | 45.778.942 | 45.778.961 | | |
| 34 | gRNA8_DMPK | Fw | AGACGCAGGGACAGGAATGG | | 45.765.441 | 45.765.460 | 276 | 0,97 |
| 35 | | Rev | GTTCCGCTTACAGCTAGTACC | | 45.765.185 | 45.765.205 | | |
| 36 | gRNA10 DMPK | Fw | TAAGTAAGGGTGTGTGTGTTGC | | 45.772.440 | 45.772.461 | 155 | 0,95 |
| 37 | | Rev | AACAAATCAGGATTCCCACCTG | | 45.772.307 | 45.772.328 | | |
| 38 | gRNA1_CNBP | Fw | GACAAGTCTATCACAAGGGACC | 3 | 129.175.970 | 129.175.991 | 132 | 1,19 |
| 39 | | Rev | TAAAAGAGGTCTTTGAGTGGCC | | 129.175.860 | 129.175.881 | | |
| 40 | gRNA2_CNBP | Fw | CAAAGATCTGACTGCAGCCATG | | 129.171.755 | 129.171.776 | 153 | 0,82 |
| 41 | | Rev | GACAAAATACCTCTATCCGAGG | | 129.171.624 | 129.171.645 | | |
| 42 | PRL | Fw | AGGGAAACGAATGCCTGATT | 6 | 22.297.096 | 22.297.115 | 120 | 1,02 |
| 43 | | Rev | GCAGGAAACACACTTCACCA | | 22.296.996 | 22.297.015 | | |

Two μg of HMW gDNA from the HEK293 cell line (extracted as described in Example 1) were directly subjected to Cas9-mediated cleavage with the selected gRNAs as described in Example 1, ending the protocol right after the cut reaction. Then, 5 ng of intact and cleaved DNA were amplified using each primer pair (10 mM each) in a separate reaction, in the presence of KAPA library Quant qPCR mix (Roche). The accumulation of PCR product was monitored by qPCR on the cut DNA sample in comparison to the intact DNA sample (Uncut DNA) **(Figure 4A).** Both cleaved and intact DNA were amplified using primers for the PRL gene (10 mM each) that was used as internal control unaffected by cutting. The qPCR reaction was performed on a QuantStudio™ 3 Real-Time PCR System (ThermoFisher Scientific) with the following program: initial denaturation at 95°C for 10' followed by 40 cycles of 95°C for 15" and 60°C for 1'.

Fold Change results were obtained using the following formula (I), which allowed to normalize the results on the efficiency of the primers (Eff.):

$$Fold\ change = \frac{Eff.\ Target\ gene\ \wedge\ (Ct\ Uncut\ DNA - Ct\ Cut\ DNA)}{Eff.\ Control\ gene\ \wedge\ (Ct\ Control\ Uncut\ DNA - Ct\ Control\ Cut\ DNA)}$$

The formula calculates the relative change (Fold Change) of detection (Ct, Cycle threshold) in Cut vs Uncut DNA of the Target gene over the level of detection of the Control gene. The formula takes into consideration also the efficiency of amplification of each primer pair (primer Eff.). Cut efficiency of each gRNA (i.e. % of cut DNA) was then determined using the following formula (II):

$$Cut\ efficiency = (1 - Fold\ change)\ x\ 100$$

Results from the DMPK gRNA. According to the above described approach, we demonstrated that all DMPK gRNAs tested, but one, had cut efficiency > 60% **(Figure 4B),** in particular cut efficiency were 80,3% (gRNA1_DMPK), 67,6% (gRNA6_DMPK), 65,6% (gRNA7_DMPK), 84,9% (gRNA8_DMPK), 37,4% (gRNA10_DMPK). The four most efficient gRNAs (1, 6, 7 and 8) were tested further via ONT sequencing as described in Example 1. The gRNAs were paired to make sure that the enriched fragment length was >3 kbp because during library preparation fragments <3 kbp are removed. Hence, gRNA 1 was paired with gRNA 8 to enrich a fragment of 6.5 kbp whereas gRNA 6 was paired to gRNA 7 to enrich a fragment of 9.6 kbp. To prevent any cut interference between gRNAs on the same end of the target, cut reactions were performed in parallel and then pooled prior to ONT sequencing. The assay was performed on gDNA extracted from the HEK293 cell line as described in the Example 1. Sequencing data analysis showed that both gRNA pairs performed clear cuts on both sides of the target **(Figure 2A).** However, we observed a significant difference in the cut efficiency. In agreement with qPCR results, gRNA pair SEQ ID NO: 1-8 produced significantly higher on-target reads (385, 1,833-fold enrichment) as compared to gRNA pair 6-7 (40, 190-fold enrichment) **(Figure 2B-C).** Based on these data, we could determine that > 70% cut efficiency threshold indicated best-performing gRNAs.

Results from the CNBP gRNA. According to the above described approach based on qPCR analysis, the CNBP gRNAs displayed 74,4% (gRNA1_CNBP) and 84,4% (gRNA2_CNBP) **(Figure 4C).** Since both gRNAs showed >70% cut efficiency, based on previous tests on DMPK, we concluded that these gRNAs had optimal efficiency for targeted ONT-sequencing. Confirmation of gRNA cut efficiency was performed by Cas9-mediated ONT sequencing starting from genomic DNA from the HEK293 cell line and it has been described in the Example 1. Sequencing data analysis showed that the gRNA pair performed clear cuts on both sides of the target **(Figure 3A)** and confirmed the good cut efficiency of CNBP gRNAs identified based on qPCR analysis, with 1,170 (1.8%) reads mapping on the CNBP microsatellite and 5,571-fold enrichment **(Figure 3B-C).**

**Claims**

**1.** An in-vitro method for diagnosing a repeat expansion-linked disorder in a human subject, said method comprising the steps of:

a. providing a biological sample comprising genomic DNA from said subject;
b. extracting genomic DNA from the biological sample, preferably said genomic DNA is high-molecular-weight DNA (>50kbp);
c. analysing the portion of the genomic DNA in said sample by Cas9-mediated DNA enrichment and by long-read DNA sequencing,

wherein said repeat expansion- linked disorder is chosen from the group consisting of Myotonic dystrophy type 1 (MD1) and Myotonic dystrophy type 2 (MD2), and wherein said method allows to determine whether said subject is suffering from or has a genetic predisposition to develop Myotonic dystrophy type 1 or from Myotonic dystrophy type 2.

**2.** The in-vitro method according to claim 1, wherein said step of long-read DNA sequencing is carried out by nanopore sequencing (i.e. ONT-sequencing) or SMRT sequencing (i.e. PacBio) or other long-read sequencing technologies at single nucleotide resolution.

**3.** The in-vitro method according to any one of claims 1 or 2, wherein said Myotonic dystrophy type 1 is due to a nucleotide expansion within the DMPK (DM1 protein kinase) gene, whereas said Myotonic dystrophy type 2 is due to a nucleotide expansion within the CNBP (CCHC-type zinc finger nucleic acid binding protein) gene.

**4.** The in-vitro method according to any one of claims 1 to 3, wherein said method is a PCR-free method.

**5.** The in-vitro method according to any one of claims 1 to 4, wherein said biological sample comprising genomic DNA is chosen from the group consisting of fetal cells obtained by amniocentesis, fetal cells obtained by chorionic villus sampling, biopsy (eg. from muscle, nerve, bone marrow), blood, plasma, serum, urine, saliva and tears.

**6.** The in-vitro method according to any one of claims 1 to 5, wherein said step c. of analysing the portion of the genomic DNA in the sample by Cas9-mediated DNA enrichment and by long-read DNA sequencing is carried out by focusing on the portion of the genomic DNA in the sample corresponding to the DMPK gene on chromosome 19q13.32 and determining the presence or absence of a CTG repeat expansion in the 3'-UTR of the DMPK gene, wherein a determination of the absence of a CTG repeat expansion in the 3'-UTR of the DMPK gene indicates that the subject does not have a genetic predisposition to develop, and is not suffering from Myotonic Dystrophy type 1, and/or wherein a determination of the presence of a CTG repeat expansion in the 3'-UTR of the DMPK gene indicates that the subject has a genetic predisposition to develop, or is suffering from Myotonic Dystrophy type 1 (MD1).

**7.** The in-vitro method according to any one of claims 1 to 5, wherein said step c. of analysing the portion of the genomic DNA in the sample by Cas9-mediated DNA enrichment and by long-read DNA sequencing is carried out by focusing on the portion of the genomic DNA in the sample corresponding to the CNBP gene on chromosome 3q21.3 and determining the presence or absence of a CCTG or TCTG repeat expansion in the intron 1 of the CNBP gene, wherein a determination of the absence of a CCTG or TCTG repeat expansion in the intron 1 of the CNBP gene indicates that the subject does not have a genetic predisposition to develop, and is not suffering from Myotonic Dystrophy type 2, and/or wherein a determination of the presence of a CCTG or TCTG repeat expansion in the 3'-UTR of the CNBP gene indicates that the subject has a genetic predisposition to develop, or is suffering from Myotonic Dystrophy type 2 (MD2).

**8.** The in-vitro method according to any one of claims 1 to 7, wherein said step c. of analysing the portion of the genomic DNA in the sample by Cas9-mediated sequencing comprises contacting the sample with a guideRNA that specifically hybridizes to at least a portion of the region corresponding to nucleotides chr19:45,745,000-45,799,000 (hg38), which encompasses a portion of the DMPK locus in the 19q13.32 band.

**9.** The in-vitro method according to claim 8, wherein said step c. of analysing the portion of the genomic DNA in the sample by Cas9-mediated DNA enrichment comprises contacting the sample with the guideRNA selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10.

**10.** The in-vitro method according to any one of claims 1 to 7, wherein said step c. of analysing the portion of the genomic DNA in the sample by Cas9-mediated sequencing comprises contacting the sample with a guideRNA that specifically hybridizes to at least a portion of the region corresponding to nucleotides chr3:129,163,000-129,185,000 (hg38), which encompasses a portion of the CNBP locus in the 3q21.3 band.

**11.** The in-vitro method according to claim 10, wherein said step c. of analysing the portion of the genomic DNA in the sample by Cas9-mediated DNA enrichment comprises contacting the sample with the guideRNA selected from the group consisting of: SEQ ID NO: 11 and SEQ ID NO: 12.

**12.** Use of an isolated oligonucleotide guideRNA for diagnosing a repeat expansion-linked disorder chosen from the group consisting of Myotonic dystrophy type 1 and Myotonic dystrophy type 2 in a human subject, wherein:

MD1 is diagnosed by analysing the portion of the genomic DNA in the sample corresponding to the DMPK gene on chromosome 19q13.32 and determining the presence or absence of a CTG repeat expansion in the 3'-UTR of the DMPK gene with at least one guideRNA chosen from the group consisting of SEQ ID NO: 1, SEQ ID NO:

2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10; and

MD2 is diagnosed by analysing the portion of the genomic DNA in the sample corresponding to the CNBP gene on chromosome 3q21.3 and determining the presence or absence of a CCTG or TCTG repeat expansion in the intron 1 of the CNBP gene with at least one guideRNA chosen from the group consisting of SEQ ID NO: 11 and SEQ ID NO: 12.

13. A kit for diagnosing a repeat expansion- linked disorder chosen from the group consisting of Myotonic dystrophy type 1 and Myotonic dystrophy type 2 in a human subject, said kit comprising:

at least one guideRNA chosen from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO:10; and/or
at least one guideRNA chosen from the group consisting of SEQ ID NO: 11 and SEQ ID NO: 12,
said kit further comprising instructions for use in the method of claims 1-12.

14. A method for the validation of the cut efficiency a guideRNA comprising the steps of:

a. selecting a guideRNA to be validated;
b. subjecting a sample of genomic DNA to Cas9-mediated cleavage using the selected guideRNA of step a., to obtain a cleaved DNA (Cut DNA);
c. amplifying the cleaved DNA (Cut DNA) of step b. with a primer pair surrounding the gRNA cut site (Target gene) and in parallel amplifying the cleaved DNA (Cut DNA) of step b. with a second primer pair amplifying a genomic region unaffected by Cas9 cleavage (Control Cut DNA);
d. amplifying a sample of genomic DNA (Uncut DNA) with the primer pair surrounding the gRNA cut site (Target gene) of step b. and in parallel amplifying a sample of genomic DNA (Uncut DNA) with the second primer pair of step b. (Control Uncut DNA);
e. validating the cut efficiency of the guideRNA by applying formula (I):

$$Fold\ change = \frac{Eff.\ Target\ gene\ {}^\wedge (Ct\ Uncut\ DNA - Ct\ Cut\ DNA)}{Eff.\ Control\ gene\ {}^\wedge (Ct\ Control\ Uncut\ DNA - Ct\ Control\ Cut\ DNA)}$$

wherein the Cut efficiency of each gRNA (i.e. % of cut DNA) is determined using formula (II):

$$Cut\ efficiency = (1 - Fold\ change)\ x\ 100$$

wherein a cut efficiency value of more than or equal to 70% is indicative that the selected guideRNA has been validated.

**Figure 1.**

EP 4 276 195 A1

**Figure 2.**

A  Hg38_DMPK_Chr19

B  Coverage

C  Enrichment

Figure 3.

**A** Hg38_CNBP_Chr3

EP 4 276 195 A1

Figure 4.

Europäisches
Patentamt

European
Patent Office

Office européen
des brevets

# EUROPEAN SEARCH REPORT

**Application Number**

**EP 22 17 2653**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/014409 A1 (ILLUMINA INC [US]) 28 January 2016 (2016-01-28) | 1,4,5 | INV. C12Q1/6827 |
| Y | * abstract * * paragraphs [0002], [0163], [0181] * * claims 1,21,48,149,153 * | 2,3,6-11 | C12Q1/6883 |
| X | WO 2015/075056 A1 (THERMO FISHER SCIENTIFIC BALTICS UAB [LT]) 28 May 2015 (2015-05-28) | 1,4,5 | |
| Y | * abstract * * page 5, line 15 - line 18 * * page 8, line 6 - line 15 * * examples 16,25 * * claims 6-12 * * figures 6-12 * | 2,3,6-11 | |
| X | WO 2018/002812 A1 (CRISPR THERAPEUTICS AG [CH]) 4 January 2018 (2018-01-04) | 12,13 | |
| Y | * abstract * * paragraph [0214] - paragraph [0215] * * paragraph [0225] - paragraph [0230] * * paragraphs [0340], [0378] * * paragraph [0420] * * paragraph [0478] - paragraph [0481] * * figures 2, 4 * * claim 54 * | 9 | |

TECHNICAL FIELDS
SEARCHED      (IPC)

C12Q

~~The present search report has been drawn up for all claims~~

| Place of search | Date of completion of the search | Examiner |
|---|---|---|
| Munich | 31 October 2022 | Barz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..............................................................................................

& : member of the same patent family, corresponding document

EPO FORM 1503 03.82 (P04C01)

Europäisches
Patentamt

European
Patent Office

Office européen
des brevets

**Application Number**

EP 22 17 2653

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1–13

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

The Search Division considers that the present European patent application does not comply with the requirements of unity of invention and relates to several inventions or groups of inventions, namely:

1. claims: 1-13

An in-vitro method for diagnosing a repeat expansion-linked disorder in a human subject, said method comprising the steps of a) providing a biological sample comprising genomic DNA from said subject, b) extracting genomic DNA from the biological sample, preferably said genomic DNA is high-molecular-weight DNA (>50kbp);c. analysing the portion of the genomic DNA in said sample by Cas9-mediated DNA enrichment and by long-read DNA sequencing, wherein said repeat expansion-linked disorder is chosen from the group consisting of Myotonic dystrophy type 1 (MD1) and Myotonic dystrophy type 2 (MD2), and wherein said method allows to determine whether said subject is suffering from or has a genetic predisposition to develop MD1 or from MD2. Use of an isolated oligonucleotide guideRNA chosen from SEQ ID NO:s 1-12 for diagnosing MD1 or MD2 in a human subject. Kit for diagnosing MD1 or MD2 in a human subject, said kit comprising at least one guideRNA chosen from SEQ ID NO:s 1-12 and further comprising instructions for use in the method of claims 1-12.

---

2. claim: 14

Method for the validation of the cut efficiency a guideRNA comprising the steps of a) selecting a guideRNA to be validated, b) subjecting a sample of genomic DNA to Cas9-mediated cleavage using the selected guideRNA of step a), to obtain a cleaved DNA (Cut DNA), c) amplifying the cleaved DNA (Cut DNA) of step b) with a primer pair surrounding the gRNA cut site (Target gene) and in parallel amplifying the cleaved DNA (Cut DNA) of step b) with a second primer pair amplifying a genomic region unaffected by Cas9 cleavage (Control Cut DNA), d) amplifying a sample of genomic DNA (Uncut DNA) with the primer pair surrounding the gRNA cut site (Target gene) of step b) and in parallel amplifying a sample of genomic DNA (Uncut DNA) with the second primer pair of step b) (Control Uncut DNA), e) validating the cut efficiency of the guideRNA by applying formula (I), wherein the Cut efficiency of each gRNA (i.e. % of cutDNA) is determined using formula (II), wherein a cut efficiency value of more than or equal to 70% is indicative that the selected guideRNA has been validated.

---

## ANNEX TO THE EUROPEAN SEARCH REPORT
## ON EUROPEAN PATENT APPLICATION NO.

EP 22 17 2653

This annex lists the patent family members relating to the patent documents cited in the above-mentioned European search report.
The members are as contained in the European Patent Office EDP file on
The European Patent Office is in no way liable for these particulars which are merely given for the purpose of information.

31-10-2022

| Patent document cited in search report | | Publication date | Patent family member(s) | | Publication date |
|---|---|---|---|---|---|
| WO 2016014409 | A1 | 28-01-2016 | AU | 2015294354 A1 | 02-02-2017 |
| | | | AU | 2021282536 A1 | 06-01-2022 |
| | | | CA | 2955382 A1 | 28-01-2016 |
| | | | CA | 3176503 A1 | 28-01-2016 |
| | | | CN | 107109401 A | 29-08-2017 |
| | | | CN | 112941065 A | 11-06-2021 |
| | | | EP | 3172321 A1 | 31-05-2017 |
| | | | SG | 11201702066U A | 27-04-2017 |
| | | | US | 2016017396 A1 | 21-01-2016 |
| | | | US | 2020165650 A1 | 28-05-2020 |
| | | | WO | 2016014409 A1 | 28-01-2016 |
| WO 2015075056 | A1 | 28-05-2015 | NONE | | |
| WO 2018002812 | A1 | 04-01-2018 | EP | 3478829 A1 | 08-05-2019 |
| | | | US | 2019211362 A1 | 11-07-2019 |
| | | | WO | 2018002812 A1 | 04-01-2018 |

For more details about this annex : see Official Journal of the European Patent Office, No. 12/82